# EUROPEAN PATENT APPLICATION

(11) **EP 1 345 151 A2**
(43) Date of publication of application: **17.09.2003**
(21) Application number: 02025420.7
(22) Date of filing: 14.11.2002
(51) Int. Cl.: G06F 19/00

(54) **Medical-information supplying method and apparatus**

(30) Priority: 12.03.2002 JP 2002066564
(71) Applicant: Colin Corporation, Komaki-shi, Aichi-ken (JP)
(72) Inventor: Oka, Tohru, Komaki-shi, Aichi-ken (JP); Narimatsu, Kiyoyuki, Komaki-shi, Aichi-ken (JP)
(74) Representative: Winter, Brandl, Fürniss, Hübner, Röss, Kaiser, Polte Partnerschaft

(57) **Abstract**

A method of supplying, from a server which is connected via a communication line to a plurality of member's terminal devices which are respectively operated by a plurality of members who are registered in advance in the server, a set of medical information corresponding to a set of physical information sent to the server from one of the member's terminal devices, operated by one of the members, to said one member's terminal device, the method including a medical-information selecting step of selecting, from a plurality of sets of medical information which correspond to a plurality of symptoms, respectively, and which are stored in advance in a medical-information data base, a set of medical information corresponding to the set of physical information sent to the server from the one member's terminal device operated by the one member, and a medical-information supplying step of supplying the selected set of medical information to the one member's terminal device of the one member.

## Description

### BACKGROUND OF THE INVENTION

### Field of the Invention

The present invention relates to a medical-information supplying method and a medical-information supplying apparatus that can perform medical consultation or examination based on physical information of a member and allow the member to obtain, as needed, more reliable medical information from a medical person such as a doctor.

### Related Art Statement

When a person takes a periodic medical examination for preventing diseases, worries about physical condition, or is actually in poor condition, the person consults a medical institution such as a hospital or a doctor's office to have a diagnosis made by a medical person such as a specialist or a practitioner.

However, in many cases, the person must wait, in the medical institution such as hospital or doctor's office, even two or three hours. Thus, the person needs a lot of time to take medical examination, or must even absent himself or herself from business. In addition, the person needs a lot of expense because the medical person such as specialist or practitioner actually examines him or her.

### SUMMARY OF THE INVENTION

It is therefore an object of the present invention to provide a medical-information supplying method and a medical-information supplying apparatus that allow a person to easily take medical examination via a communication line, without costing the person much time or expense.

The above object has been achieved by the present invention. According to a first aspect of the present invention, there is provided a method of supplying, from a server which is connected via a communication line to a plurality of member's terminal devices which are respectively operated by a plurality of members who are registered in advance in the server, a set of medical information corresponding to a set of physical information sent to the server from one of the member's terminal devices, operated by one of the members, to the one member's terminal device, the method comprising a medical-information selecting step of selecting, from a plurality of sets of medical information which correspond to a plurality of symptoms, respectively, and which are stored in advance in a medical-information data base, a set of medical information corresponding to the set of physical information sent to the server from the one member's terminal device operated by the one member, and a medical-information supplying step of supplying the selected set of medical information to the one member's terminal device of the one member.

If a living person registered as a member sends, to the server, a set of physical information via a member's terminal device, then, at the medical-information selecting step, the server selects, from the sets of medical information stored in the medical-information data base, a set of medical information corresponding to the set of physical information sent from the member and, at the medical-information supplying step, the server supplies the selected set of medical information to the member. Thus, even if the member may be a person living at a remote place, the member can automatically obtain an evaluation or a diagnosis on the set of physical information. That is, the member can easily take medical examination via the communication line, without needing much time or expense.

According to a preferred feature of the first aspect of the present invention, the medical-information supplying method further comprises a physical-information-abnormality identifying step of identifying an abnormality of the set of physical information sent from the one member, by judging whether the set of physical information falls outside a reference range, a medical person selecting step of selecting, when the abnormality of the set of physical information sent from the one member has been identified, one of a plurality of medical persons who respectively operate a plurality of medical person's terminal devices, and a sending and receiving step of sending the set of physical information whose abnormality has been identified, to the medical person's terminal device of the selected medical person, so that the selected medical person makes a diagnosis on the set of physical information, and receiving the diagnosis from the medical person's terminal device of the selected medical person, and the medical-information supplying step comprises supplying the selected set of medical information and the diagnosis received from the medical person's terminal device of the selected medical person, to the one member's terminal device of the one member.

According to this feature, when the abnormality of the set of physical information has been identified at the physical-information-abnormality identifying step, the server selects, at the medical person selecting step, one of the pre-registered medical persons who respectively operate the medical person's terminal devices and, then at the sending and receiving step, sends the set of physical information whose abnormality has been identified, to the medical person's terminal device of the selected medical person, so that the selected medical person makes a diagnosis on the set of physical information, and receives the diagnosis from the medical person's terminal device of the selected medical person. Subsequently, at the medical-information supplying step, the server supplies the diagnosis received from the medical person's terminal device of the selected medical person, to the member's terminal device of the member. Thus, when the set of physical information is abnormal, the member can obtain reliable medical information.

According to another feature of the first aspect of the present invention, the medical person selecting step comprises selecting the one medical person based on a sort of the set of physical information whose abnormality has been identified.

According to this feature, the server selects a medical person appropriate for the set of physical information judged as abnormal, for example, a specialist about the abnormal physical information. Thus, when the set of physical information is abnormal, the member can obtain more reliable medical information.

According to another feature of the first aspect of the present invention, the medical person selecting step comprises selecting the one medical person based on an address of the one member who has sent the set of physical information whose abnormality has been identified.

According to this feature, the server selects a medical person appropriate for the address of the member who has sent the set of physical information, for example, a doctor belonging to a medical institution located in an area in which the member can go from his or her home. Thus, when the set of physical information is judged as abnormal, the member can take re-examination of the doctor who has made the judgment.

According to another feature of the first aspect of the present invention, the medical-information supplying method further comprises a charging step of charging the one member when the diagnosis received from the medical person's terminal device of the selected medical person is supplied to the one member's terminal device of the one member.

According to this feature, the member who has obtained the diagnosis made by the medical person on the set of physical information judged as abnormal, i.e., the beneficiary pays the fee. Therefore, the cost needed to run the virtual-hospital service in which an evaluation or an examination corresponding to a set of physical information is automatically selected from the medical-information data base, i.e., the membership fee can be reduced. Thus, the member can take usual examination on his or her physical information, at the low fee.

According to another feature of the first aspect of the present invention, a plurality of physical-information obtaining devices are connected to the plurality of member's terminal devices, respectively, and obtain respective sets of physical information from the members and supply the respective sets of physical information to the member's terminal devices.

According to this feature, the members need not input manually the respective sets of physical information into the respective member's terminal devices. In addition, a diagnosis made based on each set of physical information can enjoy a high reliability.

According to a second aspect of the present invention, there is provided a medical-information supplying apparatus including a server which is connected via a communication line to a plurality of member's terminal devices which are respectively operated by a plurality of members who are registered in advance in the server, and supplying, from the server, a set of medical information corresponding to a set of physical information sent to the server from one of the member's terminal devices, operated by one of the members, to the one member's terminal device, the apparatus comprising a medical-information data base in which a plurality of sets of medical information respectively corresponding to a plurality of symptoms are stored; a medical-information selecting means for selecting, from the plurality of sets of medical information stored in the medical-information data base, a set of medical information corresponding to the set of physical information sent to the server from the-one member's terminal device operated by the one member; and a medical-information supplying means for supplying the selected set of medical information to the one member's terminal device of the one member.

When a living person registered as a member sends, to the server, a set of physical information via a member's terminal device, the medical-information selecting means selects, from the sets of medical information stored in the medical-information data base, a set of medical information corresponding to the set of physical information sent from the member, and the medical-information supplying means supplies the selected set of medical information to the member. Thus, even if the member may be a person living at a remote place, the member can automatically obtain an evaluation or a diagnosis on the set of physical information. That is, the member can easily take medical examination via the communication line, without needing much time or expense.

According to a preferred feature of the second aspect of the present invention, the medical-information supplying apparatus further comprises a physical-information-abnormality identifying means for identifying an abnormality of the set of physical information sent from the one member, by judging whether the set of physical information falls outside a reference range; a medical person selecting means for selecting, when the physical-information-abnormality identifying means has identified the abnormality of the set of physical information sent from the one member, one of a plurality of medical persons who respectively operate a plurality of medical person's terminal devices; and a sending and receiving means for sending the set of physical information whose abnormality has been identified by the physical-information-abnormality identifying means, to the medical person's terminal device of the medical person selected by the medical person selecting means, so that the selected medical person makes a diagnosis on the set of physical information, and receiving the diagnosis from the medical person's terminal device of the selected medical person, and the medical-information supplying means supplies the selected set of medical information and the diagnosis received by the sending and receiving means from the medical person's terminal device of the selected medical person, to the one member's terminal device of the one member.

According to this feature, when the abnormality of the set of physical information has been identified by the physical-information-abnormality identifying means, the medical person selecting means selects one of the pre-registered medical persons who respectively operate the medical person's terminal devices, and the sending and receiving means sends the set of physical information whose abnormality has been identified, to the medical person's terminal device of the selected medical person, so that the selected medical person makes a diagnosis on the set of physical information, and receives the diagnosis from the medical person's terminal device of the selected medical person. Subsequently, the medical-information supplying means supplies the diagnosis to the member's terminal device of the member. Thus, when the set of physical information is abnormal, the member can obtain reliable medical information.

According to another feature of the second aspect of the present invention, the medical person selecting means selects the one medical person based on a sort of the set of physical information whose abnormality has been identified by the physical-information- abnormality identifying means.

According to this feature, the medical person selecting means selects a medical person appropriate for the set of physical information judged as abnormal, for example, a specialist about the abnormal physical information. Thus, when the set of physical information is abnormal, the member can obtain more reliable medical information.

According to another feature of the second aspect of the present invention, the medical person selecting means selects the one medical person based on an address of the one member who has sent the set of physical information whose abnormality has been identified by the physical-information- abnormality identifying means.

According to this feature, the medical person selecting means selects a medical person appropriate for the address of the member who has sent the set of physical information, for example, a doctor belonging to a medical institution located in an area in which the member can go from his or her home. Thus, when the set of physical information is judged as abnormal, the member can take re-examination of the doctor who has made the judgment.

According to another feature of the second aspect of the present invention, the medical-information supplying apparatus further comprises a charging means for charging the one member when the diagnosis received from the medical person's terminal device of the selected medical person is supplied to the one member's terminal device of the one member.

According to this feature, the member who has obtained the diagnosis made by the medical person on the set of physical information judged as abnormal, i.e., the beneficiary pays the fee. Therefore, the cost needed to run the virtual-hospital service in which an evaluation or an examination corresponding to a set of physical information is automatically selected from the medical-information data base, i.e., the membership fee can be reduced. Thus, the member can take usual examination on his or her physical information, at the low fee.

According to another feature of the second aspect of the present invention, the medical-information supplying apparatus further comprises a plurality of physical-information obtaining devices which are connected to the member's terminal devices, respectively, and which obtain respective sets of physical information from the members and supply the respective sets of physical information to the member's terminal devices.

According to this feature, the members need not input manually the respective sets of physical information into the respective member's terminal devices. In addition, a diagnosis made based on each set of physical information can enjoy a high reliability.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a medical-information supplying system as a medical-information supplying apparatus utilizing a communication line, to which the present invention is applied;
Fig. 2 is a block diagram for explaining essential control functions of a server shown in Fig. 1;
Fig. 3 is a view showing an example of a set of medical information that is supplied from the server of Fig. 1 to a member; and
Fig. 4 is a flow chart representing the essential control functions of the server of Fig. 1, i.e., a virtual-hospital controlling routine.

### DETAILED DESCRIPTION OF PREFERRED EMBODIMENTS

Hereinafter, there will be described a preferred embodiment of the present invention in detail by reference to the drawings.

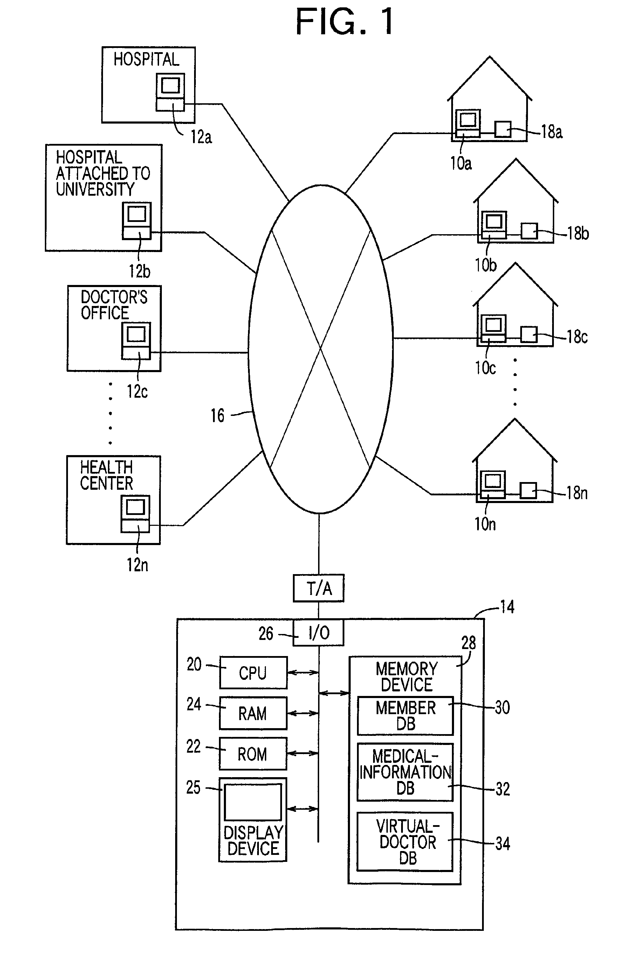

Fig. 1 is a view for explaining the construction of a medical-information supplying system as a medical-information supplying apparatus to which the present invention is applied. As shown in Fig. 1, the present medical-information supplying system includes a plurality of stationary-type or portable-type member's terminal devices 10a, 10b, 10c, ..., 10n that are operable by respective members. The member's terminal devices may be television sets, personal computers, or portable telephones that have the function of making communications via the internet. The supplying system additionally includes a plurality of medical person's terminal devices 12a, 12b, 12c, ..., 12n that are operable by respective medical persons belonging to medical institutions, such as hospitals, university's hospitals, doctor's offices, or health centers; and a server 14 as a virtual hospital that is provided in a medical-information supplying company. The supplying system further includes a communication line 16, such as wire or wireless internet (i.e., communication network) or wire or wireless telephone line, that connects the member's terminal devices 10n, the medical person's terminal devices 12n, and the server 14, with one another, so that highly secret codes, such as SSL, can be communicated among those elements 10n, 12n, 14. The member's terminal devices 10n and the medical person's terminal devices 12n may be provided by, e.g., personal computers including display devices, and the server 14 may be provided by, e.g., a high-speed and high-capacity electronic computer.

A plurality of physical-information obtaining devices 18a, 18b, 18c, ..., 18n are connected to the plurality of member's terminal devices 10a, 10b, 10c, ..., 10n, respectively. Each of the physical-information obtaining devices 18 periodically obtains, from a corresponding one of the members (i.e., living persons), various sorts of physical information including blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes. Each of the physical-information obtaining devices 18 may include a plurality of sensors that detect the above-indicated various sorts of physical information, respectively, or an input device that is manually operable by a living person to input the above-indicated various sorts of physical information. The physical-information obtaining devices 18n supply the thus obtained respective sets of physical information to the member's terminal devices 10n, respectively. The blood pressure BP, e.g., systolic and diastolic blood pressure values, may be detected in such a manner that each of the physical-information obtaining devices 18n employs an inflatable cuff, the cuff is wound around an upper arm of the corresponding living person, and a blood pressure BP of the person is determined, according to so-called oscillometric method, based on change of respective amplitudes of respective heartbeat-synchronous pulses of a cuff pulse wave detected by a pressure sensor as a pressure signal from the cuff during changing of the pressing pressure of the cuff. Alternatively, the blood pressure BP may be obtained in such a manner that blood pressure values measured using a common sphygmomanometer are inputted through operation of keys of an input device. The weight W may be detected in such a manner that each of the physical-information obtaining devices 18n employs a weight sensor, and a weight of the corresponding living person is detected by the weight sensor, or alternatively the weight W may be obtained in such a manner that a weight measured using a common scales is inputted through operation of keys of an input device. The heart rate HR may be calculated based on the number of the heartbeat-synchronous pulses of the above-described cuff pulse wave that are produced in unit time, or based on the number of heartbeat-synchronous pulses of an electrocardiograph waveform that are produced in unit time and are detected by an electrocardiograph device, not shown. The electrocardiograph waveform ECG may be detected by an electrocardiograph device, not shown. The temperature TB may be detected by a temperature sensor, not shown, that is supported by the above-described cuff and is worn with the cuff on each living person. The autonomic-nerve activity may be determined based on fluctuations of the heart rate values HR or the blood pressure values BP. The pulse-wave propagation velocity PWV may be determined based on a propagation time from the time when a second heart sound II is detected by a heart-sound microphone to the time when a dicrotic notch of a carotid pulse wave is detected, and a propagation distance between the heart and the carotid artery. The augmentation index AI may be defined as a ratio or proportion of one of an amplitude of an incident-wave component of an arterial pulse wave, such as carotid pulse wave, and an amplitude of a reflected-wave component of the pulse wave, to the other, and may be determined based on the waveform of the carotid pulse wave or the cuff pulse wave.

The server 14 is essentially provided by a so-called microcomputer including a CPU (central processing unit) 20, a ROM (read only memory) 22 that stores control programs, a RAM (random access memory) 24 that functions as a temporary-storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of a plurality of living persons who have contracted, at their own charge or no charge, with the medical-information supplying company to become, in advance, members who are supplied with medical information; respective identification codes (ID) of the members; respective pass codes or words of the members; and respective names and identification codes of the medical persons. The memory device 28 additionally includes a medical-information DB (data base) 32 that stores medical information including the respective sets of physical information sent from the members, and respective medical evaluations or diagnoses automatically made based on those sets of physical information. The memory device 28 further includes a virtual-doctor DB (data base) 34 that stores a diagnosing program for use in automatically making those medical evaluations or diagnoses based on the sets of physical information or respective changes of the sets of information.

Thus, the member's terminal devices 10n are operated by the respective members who have been registered in the server 14, and each one of the member's terminal devices 10n periodically sends a set of physical information of a corresponding one of the members to the server 14. At that time, the server 14 stores the set of physical information, and automatically makes, according to the diagnosing program pre-stored in the virtual-doctor DB 34, a medical evaluation or diagnosis based on the set of physical information or a change or trend of the set of physical information from the past sets of physical information stored in the server 14. In addition, the server 14 sends the thus made medical evaluation or diagnosis, together with the current and past sets of physical information of the member, to the member's terminal device 10n, so that the medical evaluation or diagnosis and the current and past sets of physical information are displayed on the terminal device 10n of the member. Meanwhile, each one of the medical person's terminal devices 12n is operated by a corresponding one of the medical persons who uses the pass code permitted by each one of the members to request the server 14 to send the set of medical information related to the each member. At that time, the server 14 reads, from the sets of medical information stored in the medical-information DB 32, the set of medical information related to the member corresponding to the pass code, and sends the thus obtained set of medical information to the medical person's terminal device 12n, so that the set of medical information is displayed on the terminal device 12n. The medical persons respectively operating the medical person's terminal device 12n are registered in advance in the server 14.

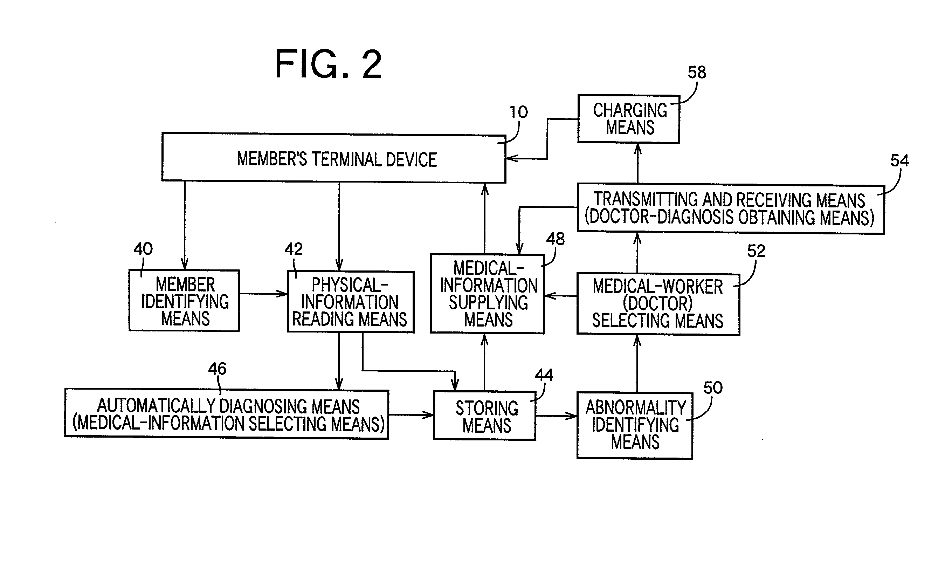

Fig. 2 is a block diagram for explaining the essential control functions of the above-described server 14. A member identifying means 40 judges whether an identification code ID inputted and sent by an arbitrary one of the member's terminal devices 10n is identical with one of the identification codes ID pre-stored in the member DB 30, and thereby judges whether a person who has made access to the server 14 is one of the members pre-registered in the server 14. A physical-information reading means 42 reads, when the member identifying means 40 has identified the person as one of the registered members, the set of physical information sent by the member's terminal device 10n, and a storing means 44 stores the thus read set of physical information in such a manner that the set of physical information is associated with the identification code ID of the identified member. A diagnosing means, i.e., a medical-information selecting means 46 automatically makes a diagnosis based on the set of physical information read by the reading means 42, according to the diagnosing program pre-stored in the virtual-doctor DB 34, and selects one of sets of medical information that corresponds to the diagnosis made. For example, the diagnosing or selecting means 46 judges whether the current set of physical information read by the reading means 42, or a change of the current and past sets of physical information, falls in a predetermined reference range, and thereby makes a medical evaluation or diagnosis, or selects a set of medical information corresponding to the medical evaluation or diagnosis made. The storing means 44 stores the thus made medical evaluation or diagnosis such that the medical evaluation or diagnosis is associated with the identification code ID or the set or sets of physical information. A medical-information supplying means 48 supplies, to the member's terminal device 10n that has sent the current set of physical information, the current and past sets of physical information, and the medical evaluation or diagnosis made on those sets of physical information, all of which have been stored by the storing means 44, so that the trend of physical information and the medical evaluation or diagnosis are displayed on the member's terminal device 10n.

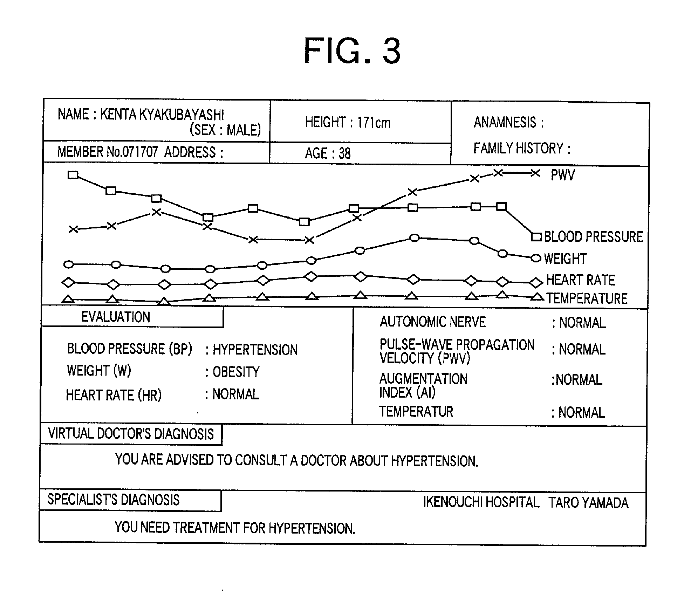

An abnormality identifying means 50 judges whether each set of physical information read by the reading means 42 falls outside a predetermined normal range and thereby judges whether each member is abnormal. The normal range may be identical with the above-described reference range. A doctor or medical person selecting means 52 selects, when the abnormality identifying means 50 judges based on the set of physical information that the member is abnormal, an appropriate one of the medical persons (e.g., doctors) pre-stored or pre-registered in the memory device 28, based on the sort of the physical information judged as abnormal, and an address of the abnormal member, such that the selected medical person or doctor is specialized in the sort of the abnormality and is near to the address of the abnormal member. A doctor-diagnosis obtaining means or a transmitting and receiving means 54 sends the physical information judged as abnormal, to the appropriate doctor selected by the doctor selecting means 52, requests the doctor to make a diagnosis about the set of physical information, and receives the diagnosis made by the doctor. The storing means 44 stores the diagnosis made by the doctor, such that the diagnosis is associated with the identification code ID or the physical information, and the medical-information supplying means 48 sends the diagnosis to the member. Fig. 3 shows an example of a set of medical information that is displayed on a display device of the member's terminal device 10n that has sent the set of physical information to the server 14. A charging means 58 charges the member as the user of the member's terminal device 10n that has sent the set of physical information to the server 14, for the use of the set of medical information. More specifically described, the charging means 58 produces data to issue a bill and sends the data to the member's terminal device 10n so that the data are displayed or outputted by the terminal device 10n.

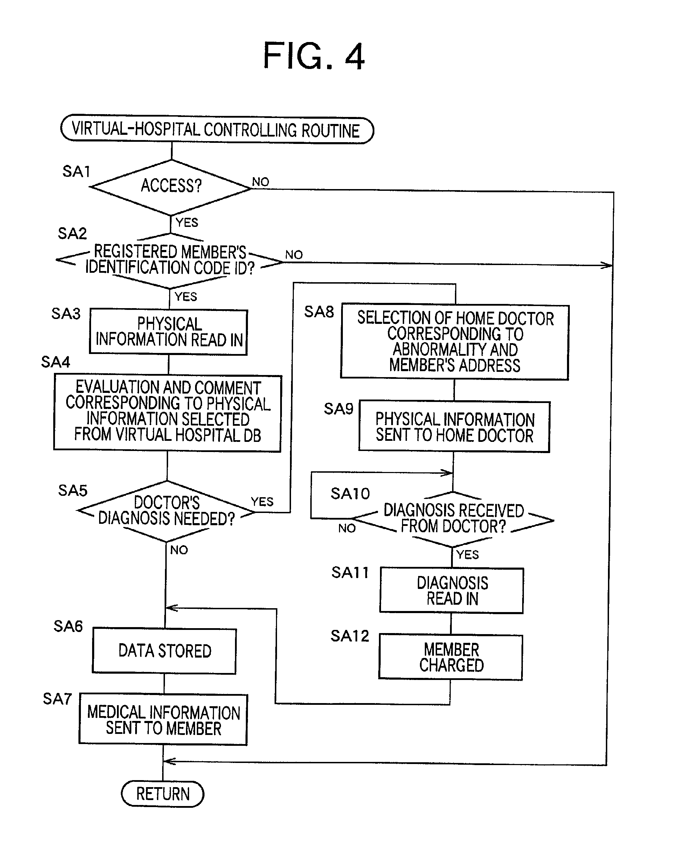

Fig. 4 shows a flow chart representing the essential control functions of the above-described server 14, i.e., a virtual-hospital controlling routine. This routine is iteratively executed at a short period of, e.g., several milliseconds.

At Step SA1 of Fig. 4 (hereinafter, "Step(s)" is omitted), the server 14 judges whether any one of the member's terminal devices 10n has made access to the server 14. If a negative judgment is made at SA1, this routine is quitted. On the other hand, if a positive judgment is made at SA1, the control goes to SA2 corresponding to the member identifying step or the member identifying means 40. At SA2, the server judges whether the member's identification code ID inputted by the member's terminal device 10n is identical with one of the identification codes ID registered in advance in the server. If a negative judgment is made at SA2, this routine is quitted. On the other hand, if a positive judgment is made at SA 2, the control goes to SA3 corresponding to the physical-information reading step or the physical-information reading means 42. At SA3, the server reads at least one set of physical information that has been obtained by the physical-information obtaining device 18n of the member's terminal device 10n and has been sent from the terminal device 10n, i.e., at least one of blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes.

Then, the control goes to SA4 corresponding to the diagnosing step or the diagnosing means, that is, the medical-information selecting step or the medical-information selecting means 46. At SA4, the server automatically makes, according to the automatically evaluating and diagnosing program stored in the virtual-doctor DB 34, a diagnosis about the new set of physical information, the past set or sets of physical information, and the change of the new and past sets of physical information. For example, the server selects one of a plurality of evaluations and/or comments that corresponds to the new set of physical information. Fig. 3 shows the thus made evaluation and the virtual doctor's diagnosis. Then, the control goes to SA5 corresponding to the abnormality identifying step or the abnormality identifying means 50. At SA5, the server judges whether it is needed to consult an actual doctor or a specialist. More specifically described, the server judges whether the new set of physical information falls outside a predetermined normal range. If a negative judgment is made at SA5, the control goes to SA6 corresponding to the storing step or the storing means 44. At SA6, the server stores, in the medical-information DB 32, the new set of physical information and the automatically made evaluation or diagnosis, such that the medical information is associated with the identification code ID of the member. Then, the control goes to SA7 corresponding to the medical-information supplying step or the medical-information supplying means 48. At SA7, the server sends, to the member's terminal device 10n that has sent the new set of physical information, a set of medical information that has been stored, for the member, in the medical-information DB 32, that is, the new and past sets of physical information (i.e., the trend of physical information) and the automatic evaluation or diagnosis, so that the set of medical information is displayed on the member's terminal device 10n as shown in Fig. 3. Thus, each member can easily and economically request the virtual hospital or doctor to check his or her physical condition or make a diagnosis on it.

On the other hand, if a positive judgment is made at SA5, the control goes to SA8 corresponding to the doctor selecting step or the doctor selecting means, i.e., medical person selecting step or medical person selecting means 52. At SA8, the server selects, from a pre-stored list of doctors, a doctor or a specialist who is appropriate for the sort of the abnormal physical information, the degree of abnormality of the physical information, and the address of the member. For example, a specialist who is specialized in the sort of the abnormal physical information, or a doctor belonging to a medical institution located in an area where the member can go. Next, the control goes to SA 9 to SA11 corresponding to the doctor-diagnosis obtaining step or the doctor-diagnosis obtaining means, i.e., transmitting and receiving step or transmitting and receiving means 54. First, at SA9, the server sends, to the selected doctor, the abnormal, new set of physical information of the member and the past sets of physical information of the same, and requests the doctor to make a diagnosis on the sets of physical information. Then, at SA10, the server judges whether the server has received a diagnosis sent from the doctor. SA10 is repeated till a positive judgment is made. When a positive judgment is made at SA10, the control goes to SA11 to read in the diagnosis sent from the doctor. Then, the control goes to SA12 as the charging step or the charging means 58. At SA12, the server charges the member who has sent the new set of physical information via the member's terminal device 10n, for the use of the diagnosis made by the doctor. Subsequently, the control goes to SA6 and SA7. At SA6, the server stores, in the medical-information DB 32, not only the new set of physical information and the automatic evaluation or diagnosis, but also the diagnosis made by the actual doctor, such that those medical information is associated with the identification code ID of the member. Then, the control goes to SA7 corresponding to the medical-information supplying step or the medical- information supplying means 48. At SA7, the server sends, to the member's terminal device 10n that has sent the new set of physical information, a set of medical information that has been stored, for the member, in the medical-information DB 32, that is, the new and past sets of physical information (i.e., the trend of physical information), the automatic evaluation or diagnosis, and the diagnosis made by the actual doctor, so that the set of medical information is displayed on the member's terminal device 10n, as shown in Fig. 3, and additionally the diagnosis made by the actual doctor, and the name and address of the hospital to which the doctor belongs, or the address of the doctor are indicated in an area prepared therefor (i.e., the lowermost area). Thus, each member can easily and economically request the virtual hospital or doctor to check his or her physical condition or make a diagnosis on it, and additionally can obtain the diagnosis made by the actual doctor when the new set of physical information is judged as abnormal. If necessary, the member can request the doctor to re-examine his or her physical condition.

As is apparent from the foregoing description of the illustrated embodiment, when a living person registered as a member sends, to the server 14, a set of physical information via a member's terminal device 10n, the medical-information selecting means 46 (SA4) selects, from the sets of medical information stored in the medical-information data base 32, a set of medical information corresponding to the set of physical information sent from the member, and the medical-information supplying means 48 (SA7) supplies the selected set of medical information to the member. Thus, even if the member may be a person living at a remote place, the member can automatically obtain an evaluation or a diagnosis on the set of physical information. That is, the member can easily take medical examination via the communication line 16, without needing much time or expense.

In addition, in the illustrated embodiment, the server 14 includes the physical-information-abnormality identifying means 50 (SA5) for judging whether the set of physical information sent from the member falls outside a reference range; the medical person selecting means 52 (SA8) for selecting, when the set of physical information sent from the member has been judged as abnormal by the physical-information-abnormality identifying means 50, one of the pre-registered medical persons who respectively operate the medical person's terminal devices 12; and the sending and receiving means 54 (SA9 through SA11) for sending the set of physical information judged as abnormal by the physical-information-abnormality identifying means 50, to the medical person's terminal device 12n of the medical person selected by the medical person selecting means 52, so that the selected medical person makes a diagnosis on the set of physical information, and receiving the diagnosis from the medical person's terminal device 12n of the selected medical person, and the medical-information supplying means 48 (SA7) supplies the diagnosis received by the sending and receiving means 54 from the medical person's terminal device 12n of the selected medical person, to the member's terminal device 10n of the member. Thus, when the set of physical information is abnormal, the member can automatically obtain, as reliable medical information, the diagnosis made by the actual medical person such as doctor.

In the illustrated embodiment, the medical person selecting means 52 (SA8) selects a medical person based on a sort of the set of physical information which has been sent from the member and whose abnormality has been identified by the physical-information-abnormality identifying means 50 (SA5). Therefore, the selecting means selects a medical person appropriate for the abnormal physical information. Thus, when the physical information is abnormal, the member can obtain more reliable medical information.

In the illustrated embodiment, the medical person selecting means 52 (SA8) selects a medical person based on an address of the member who has sent the set of physical information whose abnormality has been identified by the physical-information-abnormality identifying means 50 (SA5). Therefore, the selecting means selects a medical person appropriate for the address of the member who has sent the set of physical information, for example, a doctor belonging to a medical institution located in an area in which the member can go from his or her home. Thus, when the set of physical information is judged as abnormal, the member can take re-examination of the doctor who has made the judgment.

In the illustrated embodiment, the charging means 58 charges, when the medical-information supplying means 48 (SA7) supplies the diagnosis received from the medical person's terminal device 12n of the selected medical person to the member's terminal device 10n of the member, the member as the user of the terminal device 10n. That is, the member who has obtained the diagnosis made by the medical person on the set of physical information judged as abnormal, i.e., the beneficiary pays the fee. Therefore, the cost needed to run the virtual-hospital service in which an evaluation or an examination corresponding to a set of physical information is automatically selected from the medical-information data base, i.e., the membership fee can be reduced. Thus, the member can take usual examination on his or her physical information, at the low fee.

In the illustrated embodiment, the physical-information obtaining devices 18n are connected to the member's terminal devices 10n, respectively, and obtain respective sets of physical information from the members and supply the respective sets of physical information to the member's terminal devices. Thus, the members need not input manually the respective sets of physical information into the respective member's terminal devices 10n. In addition, a diagnosis made based on each set of physical information enjoys a high reliability.

While the present invention has been described in its embodiment by reference to the drawings, it is to be understood that the present invention can otherwise be embodied.

For example, in the illustrated embodiment, the server 14 is explained such that the server 14 is provided by a single computer. However, the server 14 may be provided by a plurality of computers, and those computers may be provided at respective positions remote from each other.

In the illustrated embodiment, each of the physical-information obtaining devices 18n connected to the member's terminal devices 10n has the functions of obtaining blood pressure BP, weight W, heart rate HR, electrocardiograph waveform ECG, temperature TB, autonomic-nerve activity, pulse-wave propagation velocity PWV, augmentation index AI, etc. However, each physical-information obtaining device 18n may be one that can obtain only a portion (one, two, ..., but not all) of the above-indicated sorts of physical information, or one that can obtain other sorts of physical information. The sorts of physical information that are not directly detected by each physical-information obtaining device 18n may be manually inputted through each member's terminal device 10n after having been measured using scales, a sphygmomanometer, a heart-rate meter, an electrocardiograph, etc. In this sense, each physical-information obtaining device 18n may be one that does not include any sensors.

In the flow chart shown in Fig. 4, the order of the steps may be changed, as needed. In addition, SA8 to SA12 of Fig. 4 may be omitted.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A method of supplying, from a server which is connected via a communication line to a plurality of member's terminal devices which are respectively operated by a plurality of members who are registered in advance in the server, a set of medical information corresponding to a set of physical information sent to the server from one of the member's terminal devices, operated by one of the members, to said one member's terminal device, the method comprising:
a medical-information selecting step of selecting, from a plurality of sets of medical information which correspond to a plurality of symptoms, respectively, and which are stored in advance in a medical-information data base, a set of medical information corresponding to the set of physical information sent to the server from said one member's terminal device operated by said one member; and
a medical-information supplying step of supplying the selected set of medical information to said one member's terminal device of said one member.

2. A method according to claim 1, further comprising:
a physical-information-abnormality identifying step of identifying an abnormality of the set of physical information sent from said one member, by judging whether the set of physical information falls outside a reference range;
a medical person selecting step of selecting, when the abnormality of the set of physical information sent from said one member has been identified, one of a plurality of medical persons who are registered in advance in the server and who respectively operate a plurality of medical person's terminal devices; and
a sending and receiving step of sending the set of physical information whose abnormality has been identified, to the medical person's terminal device of the selected medical person, so that the selected medical person makes a diagnosis on the set of physical information, and receiving the diagnosis from the medical person's terminal device of the selected medical person,
wherein the medical-information supplying step comprises supplying the selected set of medical information and the diagnosis received from the medical person's terminal device of the selected medical person, to said one member's terminal device of said one member.

3. A method according to claim 2, wherein the medical person selecting step comprises selecting said one medical person based on a sort of the set of physical information whose abnormality has been identified.

4. A method according to claim 2 or claim 3,
wherein the medical person selecting step comprises selecting said one medical person based on an address of said one member who has sent the set of physical information whose abnormality has been identified.

5. A method according to any of claims 2 to 5, further comprising a charging step of charging said one member when the diagnosis received from the medical person's terminal device of the selected medical person is supplied to said one member's terminal device of said one member.

6. A method according to any of claims 1 to 5,
wherein a plurality of physical-information obtaining devices are connected to the plurality of member's terminal devices, respectively, and obtain respective sets of physical information from the members and supply the respective sets of physical information to the member's terminal devices.

7. A medical-information supplying apparatus including a server (14) which is connected via a communication line (16) to a plurality of member's terminal devices (10) which are respectively operated by a plurality of members who are registered in advance in the server, and supplying, from the server, a set of medical information corresponding to a set of physical information sent to the server from one (10n) of the member's terminal devices, operated by one of the members, to said one member's terminal device, the apparatus comprising:
a medical-information data base (32) in which a plurality of sets of medical information respectively corresponding to a plurality of symptoms are stored;
a medical-information selecting means (42, 46) for selecting, from the plurality of sets of medical information stored in the medical-information data base (32), a set of medical information corresponding to the set of physical information sent to the server (14) from said one member's terminal device (10n) operated by said one member; and
a medical-information supplying means (44, 48) for supplying the selected set of medical information to said one member's terminal device (10n) of said one member.

8. An apparatus according to claim 7, further comprising:
a physical-information-abnormality identifying means (50) for identifying an abnormality of the set of physical information sent from said one member, by judging whether the set of physical information falls outside a reference range;
a medical person selecting means (52) for selecting, when the physical-information-abnormality identifying means has identified the abnormality of the set of physical information sent from said one member, one of a plurality of medical persons who are registered in advance in the server and who respectively operate a plurality of medical person's terminal devices; and
a sending and receiving means (54) for sending the set of physical information whose abnormality has been identified by the physical-information-abnormality identifying means, to the medical person's terminal device of the medical person selected by the medical person selecting means, so that the selected medical person makes a diagnosis on the set of physical information, and receiving the diagnosis from the medical person's terminal device of the selected medical person,
wherein the medical-information supplying means (48) supplies the selected set of medical information and the diagnosis received by the sending and receiving means from the medical person's terminal device of the selected medical person, to said one member's terminal device of said one member.

9. An apparatus according to claim 8, wherein the medical person selecting means (52) selects said one medical person based on a sort of the set of physical information whose abnormality has been identified by the physical-information-abnormality identifying means (50).

10. An apparatus according to claim 8 or claim 9,
wherein the medical person selecting means (52) selects said one medical person based on an address of said one member who has sent the set of physical information whose abnormality has been identified by the physical-information-abnormality identifying means (50).

11. An apparatus according to any of claims 8 to 10, further comprising a charging means (58) for charging said one member when the diagnosis received from the medical person's terminal device of the selected medical person is supplied to said one member's terminal device of said one member.

12. An apparatus according to any of claims 7 to 11, further comprising a plurality of physical-information obtaining devices (18) which are connected to the member's terminal devices (10), respectively, and which obtain respective sets of physical information from the members and supply the respective sets of physical information to the member's terminal devices.
